# EUROPÄISCHE PATENTANMELDUNG

(11) **EP 0 574 822 A1**
(43) Veröffentlichungstag der Anmeldung: **22.12.1993**
(21) Anmeldenummer: 93109279.5
(22) Anmeldetag: 09.06.1993
(51) Int. Cl.: A61K 31/455, A61K 9/48

(54) **Pharmazeutische Weichkapseln enthaltend Lysinclonixinat und ein Verfahren zu dessen Herstellung**

(30) Priorität: 16.06.1992 DE 4219702
(71) Anmelder: ROEMMERS S.A.I.C.F., (1230) Buenos Aires (AR)
(72) Erfinder: Vàsquez, Carlos A., Dr., 1013 Capital Federal, Buenos Aires (AR)
(74) Vertreter: von Hellfeld, Axel, Dr. Dipl.-Phys.

(57) **Zusammenfassung**

Weichgelatinekapseln mit einer Füllung aus Lysinclonixinat als Wirkstoff in einer hydrophilen Matrix. Die erfindungsgemäßen Kapseln zeichnen sich durch hervorragende Lagerstabilität, leichte Herstellbarkeit und schnelle Wirksamkeit bei oraler Verabreichung aus. Die hydrophile Matrix umfaßt Polyethylenglykol mit einem durchschnittlichen Molekulargewicht von ungefähr 200 bis 1000 oder dessen Mischung mit Propylenglykol und/oder Propylencarbonat, 3 bis 10 Gew.-% Glycerin und 5 bis 15 Gew.-% Wasser. Die Weichgelatinekapseln können bei Schmerzzuständen und Entzündungen eingesetzt werden.

## Beschreibung

Die vorliegende Erfindung betrifft eine pharmazeutische Zusammensetzung zur oralen Verabreichung, die als aktive Komponente mit analgetischer und entzündungshemmender Wirkung Lysinclonixinat enthält. Die Erfindung betrifft auch ein Verfahren zur Herstellung von Weichkapseln, die Lysinclonixinat enthalten. Weiterhin bezieht sich die Erfindung auf die Verwendung von Lysinclonixinat in Kapseln zur Behandlung von Entzündungen und Schmerzzuständen.

Das L-Lysinsalz der 2-[(3-Chloro-2-Methylphenyl)amino]-3-Pyridincarbonsäure (auch mit Lysinclonixinat bezeichnet) ist aus der DE-B-2 253 134 und der US-A-3 973 027 bekannt. Dieser Stand der Technik erwähnt Formulierungen für die orale, parenterale und rektale Verabreichung.

Die orale Verabreichung ist bei Lysinclonixinat bevorzugt. Nach dem Stand der Technik wird der Wirkstoff im wesentlichen in Form von Tabletten bereitgestellt. In dieser Form befinden sich auch Zubereitungen auf dem Markt. Es wurde jedoch beobachtet, daß bei der Verabreichung von Lysinclonixinat in Form von festen Tabletten oder pulvergefüllten Hartschalenkapseln Probleme infolge von lokalen Reizungen oder sogar Verletzungen im Verdauungstrakt auftreten. Auf der anderen Seite ist es aber von besonderer Wichtigkeit, daß der Beginn der therapeutischen Wirksamkeit nach einer oralen Verabreichung des Arzneimittels Beachtung findet: Vom Standpunkt des Patienten aus betrachtet ist es besonders wünschenswert, daß der Wirkstoff Schmerzen oder Entzündungen so schnell wie möglich zum Abklingen bringt. Aus diesem Grunde gibt es ein besonderes Bedürfnis für die Entwicklung einer oralen pharmazeutischen Zubereitung mit Lysinclonixinat, die einen beschleunigten Beginn der therapeutischen Wirksamkeit mit einer Verminderung der beobachteten körperlichen Beschwerden verbindet.

Weiche elastische Gelatinekapseln sind allgemein im Gebrauch als Alternative zu pulvergefüllten Hartschalenkapseln oder Tabletten zur oralen Verabreichung von Pharmazeutika, Vitaminen, diätetischen Mitteln und dergleichen. Weichkapseln werden von Patienten regelmäßig bevorzugt, da sie im Vergleich zu konventionellen Hartkapseln oder Tabletten leichter zu schlucken sind.

Bei einem hoch wasserlöslichen Stoff wie Lysinclonixinat begegnet die Zubereitung einer Weichgelatinekapsel einer Reihe von Problemen. Es wurde beobachtet, daß Weichkapselschalen verspröden, was Leckverluste des Füllstoffes zur Folge hatte. Daneben stellt die Oxidationsempfindlichkeit einer Füllung mit Lysinclonixinat ganz besonders bei Lösungen oder Dispersionen den Fachmann vor weitere Probleme.

Der vorliegenden Erfindung liegt dementsprechend die Aufgabe zugrunde, Lysinclonixinat in einer Verabreichungsform bereitzustellen, die eine ausgezeichnete Lagerstabilität, leichte Herstellbarkeit unter Verwendung von Standardmaschinen und schnellen Eintritt der therapeutischen Wirksamkeit nach oraler Verabreichung, ohne daß es zu Nebenwirkungen wie bei Lysinclonixinattabletten des Marktes kommt, aufweisen. Der Erfindung liegt weiterhin die Aufgabe zugrunde, ein Verfahren zur Herstellung der Verabreichungsform bereitzustellen. Darüberhinaus liegt der Erfindung die Aufgabe zugrunde, die Verwendung von Lysinclonixinat bei der Behandlung von Schmerzzuständen und Entzündungen zu ermöglichen, ohne daß die beschriebenen Nachteile auftreten.

Gegenstand der vorliegenden Erfindung ist eine Weichgelatinekapsel mit einer Füllung aus Lysinclonixinat als Wirkstoff in einer hydrophilen Matrix aus Polyethylenglykol mit einem durchschnittlichen Molekulargewicht von ungefähr 200 bis 1000 oder dessen Mischung mit Propylenglykol und/oder Propylencarbonat, 3 bis 10 Gew.-% Glycerin und 5 bis 15 Gew.-% Wasser (alle Konzentrationsangaben beziehen sich auf die Füllung). Vorzugsweise hat das Polyethylenglykol ein durchschnittliches Molekulargewicht von etwa 400. Anstelle von Polyethylenglykol können Propylenglykol oder Propylencarbonat zum Einsatz gelangen.

Die erfindungsgemäßen Weichgelatinekapseln können, falls gewünscht, Zusatzmittel wie Konservierungsstoffe, Geschmacks- und/oder Süßungsmittel enthalten. Lysinclonixinat wird in der Füllung praktisch gelöst gehalten und im Verdauungstrakt bei Auflösung der Schale der Kapsel schnell freigesetzt.

Weiterer Gegenstand der vorliegenden Erfindung ist die Füllung aus Lysinclonixinat als Wirkstoff in einer hydrophilen Matrix der zuvor beschriebenen Art für Weichgelatinekapseln.

Gegenstand der Erfindung ist auch eine pharmazeutische Verabreichungsform mit Lysinclonixinat als Wirkstoff, bestehend aus den zuvorbeschriebenen Weichgelatinekapseln mit einem Gehalt an Lysinclonixinat von 20 bis 300, vorzugsweise 125 mg.

Weiterer Gegenstand der vorliegenden Erfindung ist ein Verfahren zur Herstellung von Weichgelatinekapseln mit einer Füllung aus Lysinclonixinat, bei dem in aufeinanderfolgenden oder gleichzeitig ablaufenden Schritten die Gelatinekapseln geformt und mit einer Füllung aus Lysinclonixinat als Wirkstoff in einer hydrophilen Matrix aus Polyethylenglykol mit einem durchschnittlichen Molekulargewicht von ungefähr 200 bis 1000 oder dessen Mischung mit Propylenglykol und/oder Propylencarbonat, 3 bis 10 Gew.-% Glycerin und 5 bis 15 Gew.-% Wasser (alle Konzentrationsangaben beziehen sich auf die Füllung) gefüllt werden.

Ein weiterer Gegenstand der vorliegenden Erfindung ist die Werwendung der zuvor beschriebenen Weichgelatinekapseln als Analgetika und/oder entzündungshemmende Mittel.

Die Füllung der vorliegenden Erfindung kann zusammen mit einer konventionellen Weichgelatinekapselschale verwendet werden. Bei einer gewöhnlichen Schalenformulierung sind ungefähr 30 bis 35 Gewichtsteile Gelatine, ungefähr 15 bis 48 Gewichtsteile Weichmacher, wie Glycerin oder Sorbitol, und ungefähr 16 bis 40 Gew.-Teile Wasser enthalten. Zusätzlich kann die Gelatineschale Konservierungsmittel wie (gemischte Parabene) in geringeren Anteilen enthalten. In an sich bekannter Weise kann die Gelatinezusammensetzung gemischt und unter Vakuumbedingungen geschmolzen werden. Die Kapseln können auch zugleich gebildet und gefüllt werden unter Verwendung an sich bekannter Verfahren und Apparaturen. Die Gelatinekapseln werden in die gewünschte Form und Größe geformt, so daß sie leicht geschluckt werden können, gewöhnlicherweise mit Hilfe von Wasser. Die resultierende Kapsel ist sowohl in Wasser als auch in gastrointestinalen Flüssigkeiten löslich.

Der angegebene Bereich der durchschnittlichen Molekulargewichte (von ungefähr 200 bis 1000) für das Polyethylenglykol, das in der erfindungsgemäßen Füllung Verwendung findet, ist wesentlich, da Polyethylenglykole von geringerem Molekulargewicht dazu neigen, durch die Schalen konventioneller Weichgelatinekapseln zu diffundieren. Umgekehrt führt die Verwendung von Polyethylenglykolen mit hohem Molekulargewicht zu einer hohen Viskosität, welche die Pumpbarkeit und somit den Einsatz unter Verwendung gebräuchlicher Maschinen gefährden.

Der Gehalt an Glycerin (3 bis 10%) und Wasser (5 bis 15%) in den erfindungsgemäßen Füllungen ist wesentlich für die vorliegende Erfindung, um unerwünschte Wechselwirkungen zwischen dem Polyethylenglykol und der Weichgelatineschale zu vermindern. Glycerin und Wasser wirken als Feuchthaltemittel, indem sie eine Art Gleichgewicht zwischen dem Feuchtigkeitsgehalt der Füllung und der Schale der Weichgelatinekapsel aufrechterhalten. Gleichermaßen dient Glycerin als Gleichgewichtsweichmacher, so daß das Polyethylenglykol nicht übermäßige Mengen des Weichmachers aus der Weichgelatinekapselschale entfernt. Somit verhindern Wasser und Glycerin, daß das Polyethylenglykol die Weichgelatinekapsel hart und brüchig macht. Somit können Leckverluste und Verletzungen der Kapselhülle vermieden werden. Bevorzugte Füllungszusammensetzungen sind im folgenden Beispiel 1 angegeben.

Eine erhöhte Bioverfügbarkeit im Vergleich zu denen der Tabletten des Standes der Technik ergibt sich dadurch, daß Lysinclonixinat in den Kapselfüllungen in Lösung gehalten wird. Dadurch kann es im Verdauungstrakt schneller freigesetzt werden, wenn sich die Hülle auflöst. Diese Eigenschaft, die in dem folgenden Beispiel 2 durch pharmacokinetische Ergebnisse untermauert wird, führt zu einem schnelleren Beginn der therapeutischen Wirksamkeit und stellt einen entscheidenden Vorteil gegenüber den auf den Markt befindlichen Tabletten dar.

Die Verabreichung der Dosierungsformen der vorliegenden Erfindung hat noch einen weiteren Vorteil: Wenn sich die Hülle auflöst, diffundiert das Lysinclonixinat sehr schnell in die Umgebung. Somit kommt es weniger zu lokalen Reizungen und dem damit verbundenen Unbehagen bzw. den Verletzungen als bei gewöhnlichen Tabletten.

Erfindungsgemäße Kapseln können mit Standardmaschinen leicht hergestellt werden und sie erweisen sich auch nach längerer Lagerung unter hohen Feuchtigkeitsbedingungen als stabil, wie das Beispiel 3 zeigt.

### BEISPIEL 1

### Herstellung der Weichgelatinekapseln mit einer Füllung aus Lysinclonixinat

Es wurden Füllungen mit einer Zusammensetzung pro Kapsel gemäß der folgenden Tabelle 1 hergestellt. Das Füllungsmaterial wurde gründlich unter Stickstoffatmosphäre vermischt und in konventionellen Weichgelatinehüllen eingekapselt. Die erhaltenen Gelatinekapseln erwiesen sich als dicht und waren mit den gewöhnlichen Verarbeitungsverfahren, wie beispielsweise aus US-A-2 288 327 und US-A-2 318 718 bekannt, verträglich. Darüberhinaus erwies sich das vergelte Füllungsmaterial als temperaturstabil und entfernte nicht excessive Mengen an Wasser oder Weichmacher aus der elastischen Weichgelatinehülle.

**TABELLE 1**

| Typische Kapselfüllungszusammensetzungen (in mg/Kapsel) | | |
|---|---|---|
| Inhaltsstoff | Füllung A | Füllung B |
| Lysinclonixinat | 125 | 125 |
| Polyethylenglykol 400 | 344 | 293 |
| Wasserfreies Glycerin | 35 | 31 |
| Wasser | 56 | 50 |

### BEISPIEL 2

### Pharmacokinetische Vergleichsversuche mit Weichgelatinekapseln und Tabletten enthaltend Lysinclonixinat

Ein konventioneller pharmacokinetischer Vergleichsversuch wurde mit 28 gesunden fastenden menschlichen Freiwilligen durchgeführt. 14 von ihnen erhielten eine einzelne 125 mg Dosis einer kommerziell erhältlichen Lysinclonixinattablette, wohingegen die anderen 14 die gleiche Dosis in Form von Weichgelatinekapseln mit der Füllung A gemäß der Tabelle 1 erhielten. Die erhaltenen pharmacokinetischen Ergebnisse sind in Tabelle 2 enthalten.

**TABELLE 2**

| Pharmacokinetische Parameter der beiden pharmazeutischen Formen | | |
|---|---|---|
| Parameter | Gelatinekapseln | Tabletten |
| t_{1/2} (h) | 0,17 | 0,29 |
| tₘₐₓ (h) | 0,5 | 1,0 |
| Fläche unterhalb der Kurve (µg/h/ml) | 10,36 | 10,09 |
| Cₘₐₓ (µg/ml) | 4,8 | 5,6 |
| Bemerkungen: t_{1/2} = Halbwertszeit; tₘₐₓ = Zeit, bei der Cₘₐₓ erreicht wird; Cₘₐₓ = maximale Konzentration. | | |

### BEISPIEL 3

### Stabilitätstests mit Weichgelatinekapseln enthaltend Lysinclonixinat

Die nach dem Beispiel 1 erhaltenen Weichgelatinekapseln wurden einem längeren Stabilitätstest unterzogen. Die Testbedingungen waren 20°C bis 25°C und 50% bis 70% relative Luftfeuchtigkeit. Es zeigte sich, daß auch nach Wochen die Kapseln stabil waren, keinerlei Versprödung zeigten und der Wirkstoff sich nicht, beispielsweise durch Oxidation, verändert hatte.

## Patentansprüche

1. Weichgelatinekapseln mit einer Füllung aus Lysinclonixinat als Wirkstoff in einer hydrophilen Matrix aus
Polyethylenglykol mit einem durchschnittlichen Molekulargewicht von ungefähr 200 bis 1000 oder dessen Mischung mit Propylenglykol und/oder Propylencarbonat,
3 bis 10 Gew.-% Glycerin und
5 bis 15 Gew.-% Wasser.

2. Weichgelatinekapseln gemäß Anspruch 1, bei der das Polyethylenglykol ein durchschnittliches Molekulargewicht von 400 hat.

3. Füllung für Weichgelatinekapseln nach Maßgabe einer der vorhergehenden Ansprüche.

4. Pharmazeutische Verabreichungsform mit Lysinclonixinat als Wirkstoff, bestehend aus Weichgelatinekapseln nach einem der Ansprüche 1 oder 2, mit einem Gehalt an Lysinclonixinat von 20 bis 300 mg.

5. Verabreichungsform nach Anspruch 4 mit 125 mg Lysinclonixinat.

6. Verfahren zur Herstellung von Weichgelatinekapseln mit einer Füllung aus Lysinclonixinat, bei dem in aufeinanderfolgenden Schritten oder gleichzeitig die Gelatinekapseln geformt und mit einer Füllung aus Lysinclonixinat als Wirkstoff in einer hydrophilen Matrix aus
Polyethylenglykol mit einem durchschnittlichen Molekulargewicht von ungefähr 200 bis 1000 oder dessen Mischung mit Polyethylenglykol und/oder Propylencarbonat,
3 bis 10 Gew.-% Glycerin und
5 bis 15 Gew.-% Wasser gefüllt werden.

7. Verwendung der Weichgelatinekapseln gemäß Anspruch 1 oder 2 als Analgetika und/oder entzündungshemmende Mittel.
